# EUROPEAN PATENT SPECIFICATION

(11) **EP 3 756 731 B1**
(45) Date of publication and mention of the grant of the patent: **14.01.2026**
(21) Application number: 20182609.6
(22) Date of filing: 26.06.2020
(51) Int. Cl.: A61Q 19/08, A61K 8/365, A61K 8/64, A61K 8/34, A61K 8/37, A61K 8/39

(54) **TOPICAL COMPOSITIONS CONTAINING N-ACYL DIPEPTIDE DERIVATIVES AND GLYCOLIC ACID**
TOPISCHE ZUSAMMENSETZUNGEN MIT N-ACYLDIPEPTIDDERIVATEN UND GLYKOLSÄURE
COMPOSITIONS TOPIQUES CONTENANT DES DÉRIVÉS DE DIPEPTIDE N-ACYLE ET DE L'ACIDE GLYCOLIQUE

(30) Priority: 26.06.2019 US 201916452982
(43) Date of publication of application: 30.12.2020
(73) Proprietor: Kenvue Brands LLC, Summit, NJ 07901 (US)
(72) Inventor: BYREN, David Scott, Skillman, New Jersey 08558 (US); CHUA, Derrick, Skillman, New Jersey 08558 (US); DUFORT, Marisa DeVita, Skillman, New Jersey 08558 (US); RUSH, Allison Keene, Skillman, New Jersey 08558 (US); YANG, Jing, Skillman, New Jersey 08558 (US)
(74) Representative: Carpmaels & Ransford LLP

(56) References cited:
- EP-A1- 3 120 831
- WO-A1-2006/114657
- CA-A1- 2 853 791
- CA-C- 2 853 791
- US-A1- 2016 081 902
- US-B2- 9 067 969

## Description

The invention is defined in the appended set of claims. The present invention provides topical compositions comprising a combination of an N-acyl dipeptide derivative and (i) glycolic acid; or (ii) glyceryl dilaurate, steareth-10, and glycerin. The compositions provide enhanced penetration of the dipeptide derivative into the skin.

### BACKGROUND OF THE INVENTION

US Patent No. 9,067,969 discloses a variety of N-acyl dipeptide derivatives to treat diseases and disorders ranging from cancer and immune disorders to conditions and disorders of the cutaneous system. The '969 patent discloses a subset of N-acyl dipeptide derivatives containing valine (Val) and alanine (Ala) as preferred compounds for treating, among other things, aging-related skin changes. The '969 patent discloses a variety of administration routes for this, including topical ones. Topical compositions such as solutions, gels, lotions, creams, emulsions and the like are disclosed, and the amount of N-acyl dipeptide derivative in such compositions may range from 0.001% to 99.9 percent by weight or volume of the total composition. The patentees also disclose that these compositions may contain other cosmetic or pharmaceutical agents, including hydroxy acids such as glycolic acid, among hundreds of other agents.

Although the dipeptide derivatives of the '969 patent comprise an alkaline radical such as an amino group modified by acylation, so that they are no longer amphoteric in nature and therefore penetrate the skin more readily, improvements in their skin penetration properties are still desired.

Applicants have now discovered improved compositions and methods for increasing the penetration of N-acyl dipeptide derivatives containing Val and Ala into the skin. In particular, applicants have found that the penetration of these compounds is surprisingly increased when combined with specific amounts of glycolic acid. Accordingly, new compositions and methods utilizing a combination of N-acyl dipeptide derivatives of the formula R₁-Val-Ala-R₂ and glycolic acid are provided.

### SUMMARY OF THE INVENTION

The present invention provides a topical composition comprising an N-acyl dipeptide derivative having the formula:

R₁-Val-Ala-R₂

or an isomer or salt thereof, wherein Val is valine, Ala is alanine, R₁ is an acyl radical having up to 19 carbon atoms; R₂ is OR₃, NHR₄, or NHNHR₅; R₃ is H, an alkyl, aralkyl, or aryl radical having up to 19 carbon atoms; and R₄ and R₅ are each independently H, OH, an alkyl, aralkyl, aryl, or acyl radical having up to 19 carbon atoms, and:
(i) glycolic acid, wherein the composition comprises up to 10 weight percent of glycolic acid; or
(ii) a cosmetically acceptable topical carrier comprising glyceryl dilaurate, steareth-10, and glycerin.

The present invention also provides a non-therapeutic method of treating signs of skin aging, comprising topically applying to skin in need of treatment for at least one sign of skin aging a topical composition comprising an N-acyl dipeptide derivative having the formula:

R₁-Val-Ala-R₂

or an isomer or salt thereof, wherein Val is valine, Ala is alanine, R₁ is an acyl radical having up to 19 carbon atoms; R₂ is OR₃, NHR₄, or NHNHR₅; R₃ is H, an alkyl, aralkyl, or aryl radical having up to 19 carbon atoms; and R₄ and R₅ are each independently H, OH, an alkyl, aralkyl, aryl, or acyl radical having up to 19 carbon atoms, and glycolic acid, wherein the composition comprises up to 10 weight percent of glycolic acid.

### DETAILED DESCRIPTION OF THE INVENTION

Unless defined otherwise, all technical and scientific terms used herein have the meaning commonly understood by one of ordinary skill in the art to which the invention pertains.

As used herein, "topically applying" means directly laying on or spreading on outer skin, the scalp, or hair, e.g., by use of the hands or an applicator such as a wipe, roller, or spray.

As used herein, "cosmetically acceptable" means the ingredients the term describes are suitable for use in contact with tissues (e.g., the skin or hair) without undue toxicity, incompatibility, instability, irritation or allergic response.

As used herein, a "cosmetically acceptable active agent" is a compound (synthetic or natural) that has a cosmetic or therapeutic effect on the skin or hair.

Compositions of the present invention are suitable for treating signs of skin aging. As used herein, "signs of skin aging" includes the presence of lines including fine lines and wrinkles, loss of elasticity, uneven skin, blotchiness, diminished skin thickness, and abnormal or diminished synthesis of collagen, glycosaminoglycans, proteoglycans, elastin, or glycoproteins including fibronectin. In one embodiment, the sign of aging is selected from the presence of lines, fine lines, wrinkles, loss of elasticity, and abnormal or diminished synthesis of collagen, glycosaminoglycans, proteoglycans, elastin, or glycoproteins including fibronectin.

As used herein, "treatment" or "treating" means the amelioration, prophylaxis, or reversal of a condition, disease, or disorder, or at least one discernible symptom thereof. In one embodiment, "treatment" or "treating" refers to an amelioration, prophylaxis, or reversal of at least one measurable physical parameter related to the condition, disease, or disorder being treated, not necessarily discernible in or by the subject being treated. In another embodiment, "treatment" or "treating" refers to inhibiting or slowing the progression of a condition, disease, or disorder, either physically, e.g., stabilization of a discernible symptom, physiologically, e.g., stabilization of a physical parameter, or both. In another embodiment, "treatment" or "treating" refers to delaying the onset of a condition, disease, or disorder.

In certain embodiments, a composition of the invention is administered as a preventative measure. As used herein, "prevention" or "preventing" refers to a reduction of the risk of acquiring a given condition, disease, or disorder.

More broadly, the compositions of the invention may also be used to treat or prevent cosmetic, dermatological, or other conditions and disorders including, but not limited to, infections, deranged or disordered cutaneous or mucocutaneous tissue relevant to skin, nail and hair; oral, vaginal and anal mucosa; disturbed keratinization; inflammation; changes associated with intrinsic and extrinsic aging, and others which may or may not be related to cutaneous system. The manifestations include, but are not limited to, oily skin; acne; rosacea; age spots; blemished skin; blotches; cellulite; dermatoses; dermatitis; skin, nail and hair infections; dandruff; dryness or looseness of skin, nail and hair; xerosis; inflammation, or eczema; elastosis; herpes; hyperkeratosis; hyperpigmented skin; ichthyosis; keratoses; lentigines; melasmas; mottled skin; pseudofolliculitis barbae; photoaging and photodamage; pruritus; psoriasis; skin lines; stretch marks; thinning of skin, nail plate and hair; warts; wrinkles; oral or gum disease; irritated, inflamed, red, unhealthy, damaged or abnormal mucosa, skin, hair, nail, nostril, ear canal, anal or vaginal conditions; breakdown, defective synthesis or repair of dermal components; abnormal or diminished synthesis of collagen, glycosaminoglycans, proteoglycans and elastin, as well as diminished levels of such components in the dermis; uneven skin tone; uneven and rough surface of skin, nail and hair; loss or reduction of skin, nail and hair resiliency, elasticity and recoilability; laxity; lack of skin, nail and hair lubricants and luster; fragility and splitting of nail and hair; yellowing skin; reactive, irritating or telangiectatic skin; and dull and older-looking skin, nail and hair. In addition, the compositions of the current invention can be used for general care of skin, nail and hair; to improve skin texture and pores, flakiness and redness; to make skin soft, smooth, fresh, balanced, visibly clear, even-toned and brighter; to increase skin fullness and plumpness; and for skin bleach and lightening and wound healing; to reduce or prevent sweating or perspiration of underarm, crotch, palm, or other parts of the body.

As used herein, the term "subject" means any animal, preferably a mammal, most preferably a human, to whom a composition of the invention will be or has been administered. The term "mammal" as used herein, encompasses any mammal. Examples of mammals include, but are not limited to, cows, horses, sheep, pigs, cats, dogs, mice, rats, rabbits, guinea pigs, monkeys, and humans. In a preferred embodiment, the subject is a human.

As used herein, "wrinkle" includes fine lines, fine wrinkles, or coarse wrinkles. Examples of wrinkles include, but are not limited to, fine lines around the eyes (e.g., "crow's feet"), forehead and cheek wrinkles, frown-lines, and laugh-lines around the mouth.

As used herein, "loss of elasticity" includes loss of elasticity or structural integrity of the skin or tissue, including but not limited to sagging, lax and loose tissue. The loss of elasticity or tissue structure integrity may be a result of a number of factors, including but not limited to disease, aging, hormonal changes, mechanical trauma, environmental damage, or the result of an application of products, such as a cosmetics or pharmaceuticals, to the tissue.

As used herein, "uneven skin" means a condition of the skin associated with diffuse or mottled pigmentation, which may be classified as hyperpigmentation, such as post-inflammatory hyperpigmentation.

As used herein, "blotchiness" means a condition of the skin associated with redness or erythema.

As used herein, "cosmetic" refers to a beautifying substance or preparation which preserves, restores, bestows, simulates, or enhances the appearance of bodily beauty or appears to enhance the beauty or youthfulness, specifically as it relates to the appearance of tissue or skin.

As used herein, "cosmetically effective amount" means an amount sufficient for treating or preventing one or more signs of skin aging, but low enough to avoid serious side effects. The cosmetically effective amount of the compound or composition will vary with the particular condition being treated, the age and physical condition of the end user, the severity of the condition being treated/prevented, the duration of the treatment, the nature of other treatments, the specific compound or product/composition employed, and the particular cosmetically-acceptable carrier.

Unless otherwise indicated, a percentage or concentration refers to a percentage or concentration by weight (i.e., % (W/W). Unless stated otherwise, all ranges are inclusive of the endpoints, e.g., "from 4 to 9" includes the endpoints 4 and 9.

### N-acyl Dipeptide Derivatives

The composition comprises one or more N-acyl dipeptide derivatives having the formula:

R₁-Val-Ala-R₂

or isomers or salts thereof, wherein Val is valine, Ala is alanine, R₁ is an acyl radical having up to 19 carbon atoms; R₂ is OR₃, NHR₄, or NHNHR₅; R₃ is H, an alkyl, aralkyl, or aryl radical having up to 19 carbon atoms; and R₄ and R₅ are each independently H, OH, an alkyl, aralkyl, aryl, or acyl radical having up to 19 carbon atoms. Mixtures of the foregoing may be used.

R₁ may be an acyl radical having up to 10 carbon atoms, for example up to 8 carbon atoms, for example up to 5 carbon atoms, for example up to 4 carbon atoms, for example up to 3 carbon atoms, for example 2 or 3 carbon atoms, for example 2 carbon atoms.

R₂ may be OR₃ or NHR₄. For example, R₂ may be OR₃ or NHR₄; R₃ may be H; and R₄ may be H or OH. For example, R₂ may be NHR₄; and R₄ may be H.

In one embodiment, the dipeptide derivative is selected from the group consisting of N-Ac-Val-Ala-NH₂, N-Ac-Val-Ala-OH, N-Ac-Val-Ala-NHOH, N-Pr-Val-Ala-NH₂, and N-Pr-Val-Ala-OH, wherein Ac is acetyl and Pr is propanoyl.

In another embodiment, the dipeptide derivative is N-Ac-Val-Ala-NH₂ wherein Ac is acetyl (N-acyl-L-valine-L-alaninamide).

The N-acyl dipeptide derivative may be made by conventional synthesis methods, as known in the art.

The amount of N-acyl dipeptide derivative in the composition may be at least about 0.001, for example at least 0.01, for example at least about 0.1, for example at least about 0.2, for example at least about 0.3, for example at least about 0.4 weight percent based on the total weight of the composition.

The amount of N-acyl dipeptide derivative in the composition may be about 90 weight percent or less, for example about 50 or less, for example about 20 or less, for example about 10 or less, for example 2 or less, for example about 1 or less, for example about 0.9 or less, for example about 0.8 or less, for example about 0.7 or less, for example about 0.6 weight percent or less based on the total weight of the composition.

The amount of N-acyl dipeptide derivative in the composition may for example range from about 0.001 to about 90 weight percent based on the total weight of the composition.

In one embodiment, the amount of N-acyl dipeptide derivative in the composition is 0.01 to 2 weight percent based on the total weight in the composition. In a further embodiment, the amount of N-acyl dipeptide derivative in the composition is 0.1 to 1 weight percent based on the total weight of the composition. In another embodiment, the amount of N-acyl dipeptide derivative in the composition is about 0.5 weight percent based on the total weight of the composition.

In one embodiment, the dipeptide derivative is N-Ac-Val-Ala-NH₂ and the composition contains about 0.1 to about 1, preferably about 0.5, weight percent of N-Ac-Val-Ala-NH₂ based on the total weight of the composition.

### Glycolic Acid

The composition as defined in any one of claims 1-10 and 12-13 also contains glycolic acid. The amount of glycolic acid in the composition is 10 weight percent or less based on the total weight of the composition.

The amount of glycolic acid in the composition may be 8 weight percent or less, for example about 7 or less, for example about 6 or less, for example about 5 or less, for example about 4 weight percent or less, based on the total weight of the composition.

The amount of glycolic acid in the composition may be 1 weight percent or more, based on the total weight of the composition.

In one embodiment, the composition contains 1 to 8 weight percent of glycolic acid based on the total weight of the composition.

In another embodiment, the composition contains 1 to about 4 weight percent of glycolic acid based on the total weight of the composition.

Glycolic acid is commercially available from a variety of sources, for example as GLYPURE, a 70 weight percent solution of glycolic acid in water from DuPont.

Accordingly, the composition may contain about 0.001 to about 90 weight percent of N-acyl dipeptide derivative and 1 to 8 weight percent of glycolic acid based on the total weight of the composition. For example, the composition may contain 0.01 to 2 weight percent of N-acyl dipeptide derivative and 1 to about 4 weight percent of glycolic acid based on the total weight of the composition. For example, the composition may contain about 0.5 weight percent of N-acyl dipeptide derivative and about 1 to about 4 weight percent of glycolic acid based on the total weight of the composition.

### Other Cosmetically Acceptable Active Agents

The composition may contain one or more other cosmetically acceptable active agents.

Cosmetically acceptable active agents include for example anti-acne agents, shine control agents, anti-microbial agents, anti-inflammatory agents, anti-mycotic agents, anti-parasite agents, external analgesics, sunscreens, photoprotectors, antioxidants, keratolytic agents, surfactants, moisturizers, nutrients, vitamins, energy enhancers, anti-perspiration agents, astringents, deodorants, firming agents, anti-callous agents, and agents for hair and/or skin conditioning.

The amount of other cosmetically active agent in the composition may range from about 0.001% to about 20% by weight of the composition, e.g., about 0.005% to about 10% by weight of the composition, such as about 0.01% to about 5% by weight of the composition, based on the total weight of the composition.

The cosmetically acceptable active agent may be selected for instance from other alpha hydroxy acids, polyhydroxy acids, other dipeptides, tripeptides, benzoyl peroxide, D-panthenol carotenoids, retinoids such as retinol and retinyl palmitate, ceramides, polyunsaturated fatty acids, essential fatty acids, enzymes such as laccase, enzyme inhibitors, minerals, hormones such as estrogens, steroids such as hydrocortisone, 2-dimethylaminoethanol, copper salts such as copper chloride, peptides such as argireline and syn-ake (such as SYN^{®}-AKE, available from DSM, which comprises glycerin, aqua and dipeptide diaminobutyroyl benzylamide diacetate), those containing copper, coenzyme Q10, amino acids such as proline, vitamins, lactobionic acid, acetyl-coenzyme A, niacin, riboflavin, thiamin, ribose, electron transporters such as NADH and FADH2, natural extracts such as those from aloe vera, feverfew, oatmeal, dill, blackberry, princess tree, lemon aspen, resorcinols such as 4-hexyl resorcinol, curcuminoids, sugar amines such as N-acetyl glucosamine, and derivatives and mixtures thereof.

Examples of vitamins include, but are not limited to, vitamin A, vitamin B's such as vitamin B3, vitamin B5, and vitamin B12, vitamin C, vitamin K, and different forms of vitamin E like alpha, beta, gamma or delta tocopherols or their mixtures, and derivatives thereof.

Examples of other hydroxy acids include, but are not limited, to lactic acid, malic acid, salicylic acid, citric acid, and tartaric acid.

Examples of antioxidants include, but are not limited to, water-soluble antioxidants such as sulfhydryl compounds and their derivatives (e.g., sodium metabisulfite and N-acyl-cysteine), lipoic acid and dihydrolipoic acid, resveratrol, lactoferrin, and ascorbic acid and ascorbic acid derivatives (e.g., ascorbyl palmitate and ascorbyl polypeptide). Oil-soluble antioxidants suitable for use in the compositions of this invention include, but are not limited to, butylated hydroxytoluene, retinoids (e.g., retinol and retinyl palmitate), tocopherols (e.g., tocopherol acetate), tocotrienols, and ubiquinone. Natural extracts containing antioxidants suitable for use in the compositions of this invention, include, but not limited to, extracts containing flavonoids and isoflavonoids and their derivatives (e.g., genistein and diadzein), and extracts containing resveratrol. Examples of such natural extracts include grape seed, green tea, pine bark, and propolis.

### Topical Compositions

The compositions of the present invention are applied topically to skin or hair. Accordingly, the composition as defined in any one of claims 1-5 and 12-13 may further include a cosmetically acceptable topical carrier. The composition as defined in any one of claims 6-11 contains a cosmetically acceptable topical carrier. The carrier may make up from about 25% to about 99.99%, by weight, of the composition (e.g., from about 80% to about 99%, by weight, of the composition). In a preferred embodiment of the invention, the cosmetically acceptable topical carrier includes water.

In one embodiment of the invention as defined in any one of claims 1-6, 8, 10 and 12-13, the carrier comprises one or more of glyceryl dilaurate, steareth-10, and glycerin. The composition as defined in any one of claims 7 and 9 contains a cosmetically acceptable topical carrier comprising one or more of glyceryl dilaurate, steareth-10, and glycerin. The composition as defined in claim 11 contains a carrier comprising glyceryl dilaurate, steareth-10 and glycerin.

The composition may contain about 0.1 to about 2, for example about 0.5 to about 1.5, for example about 1 weight percent of glyceryl dilaurate based on the total weight of the composition.

The composition may contain about 0.05 to about 1, for example about 0.1 to about 0.7, for example about 0.5 weight percent of steareth-10 based on the total weight of the composition.

The composition may contain about 0.05 to about 1, for example about 0.1 to about 0.7, for example about 0.5 weight percent of glycerin based on the total weight of the composition.

The carrier may comprise water and further comprise one or more of glyceryl dilaurate, steareth-10, and glycerin.

The compositions may be made into a wide variety of product types that include but are not limited to lotions, creams, gels, sticks, sprays, ointments, cleansing liquid washes and solid bars, shampoos and hair conditioners, hair fixers, pastes, foams, powders, mousses, shaving creams, wipes, patches, hydrogels, film-forming products, facial masks and skin masks, films and make-up such as foundations, and mascaras. These product types may contain a variety of cosmetically acceptable topical carriers including, but not limited to solutions, suspensions, emulsions such as microemulsions and nanoemulsions, gels, solids and liposomes. The following are non-limiting examples of such carriers. Other carriers can be formulated by those of ordinary skill in the art.

The compositions useful in the present invention can be formulated as solutions. Solutions typically include an aqueous or organic solvent (e.g., from about 50% to about 99.99% or from about 90% to about 99% of a cosmetically acceptable aqueous or organic solvent). Examples of suitable organic solvents include propylene glycol, polyethylene glycol, polypropylene glycol, glycerol, 1,2,4-butanetriol, sorbitol esters, 1,2,6-hexanetriol, ethanol, and mixtures thereof.

Compositions useful in the subject invention may be formulated as a solution comprising an emollient. Such compositions preferably contain from about 2% to about 50% of an emollient(s). As used herein, "emollients" refer to materials used for the prevention or relief of dryness, such as by preventing the transepidermal loss of water from the skin. Examples of emollients include, but are not limited to, those set forth in the International Cosmetic Ingredient Dictionary and Handbook, eds. Pepe, Wenninger and McEwen, pp. 2930-36 (The Cosmetic, Toiletry, and Fragrance Assoc., Washington, D.C., 9th Edition, 2002) (hereinafter "ICI Handbook"). Examples of particularly suitable emollients include vegetable oils, mineral oils, and fatty esters.

A lotion can be made from such a solution. Lotions typically contain from about 1% to about 20% (e.g., from about 5% to about 10%) of an emollient(s) and from about 25% to about 90% (e.g., from about 60% to about 80%) of water.

Another type of product that may be formulated from a solution is a cream. A cream typically contains from about 5% to about 50% (e.g., from about 10% to about 20%) of an emollient(s) and from about 25% to about 85% (e.g., from about 50% to about 75%) of water.

The composition of the present invention may include water or alternatively be anhydrous or be an ointment that includes no water but organic and/or silicone solvents, oils, lipids and waxes. An ointment may contain a simple base of animal or vegetable oils or semi-solid hydrocarbons. An ointment may contain from about 2% to about 10% of an emollient(s) plus from about 0.1% to about 2% of a thickening agent(s). Examples of thickening agents include, but are not limited to, those set forth in the ICI Handbook pp. 2979-84.

The composition may be formulated as an emulsion. If the topical carrier is an emulsion, from about 1% to about 10% (e.g., from about 2% to about 5%) of the topical carrier contains an emulsifier(s). Emulsifiers may be nonionic, anionic or cationic. Examples of emulsifiers include, but are not limited to, those set forth in the ICI Handbook, pp.2962-71.

Lotions and creams can be formulated as emulsions. Typically such lotions contain from 0.5% to about 5% of an emulsifier(s). Such creams typically contain from about 1% to about 20% (e.g., from about 5% to about 10%) of an emollient(s); from about 20% to about 80% (e.g., from 30% to about 70%) of water; and from about 1% to about 10% (e.g., from about 2% to about 5%) of an emulsifier(s).

Single emulsion skin care preparations, such as lotions and creams, of the oil-in-water type and water-in-oil type are well-known in the cosmetic art and are useful in the subject invention. Multiphase emulsion compositions, such as the water-in-oil-in-water type or the oil-in-water-in-oil type, are also useful in the subject invention. In general, such single or multiphase emulsions contain water, emollients, and emulsifiers as essential ingredients.

The compositions of this invention can also be formulated as a gel (e.g., an aqueous, alcohol, alcohol/water, or oil gel using a suitable gelling agent(s)). Suitable gelling agents for aqueous and/or alcoholic gels include, but are not limited to, natural gums, acrylic acid and acrylate polymers and copolymers, and cellulose derivatives (e.g., hydroxymethyl cellulose and hydroxypropyl cellulose). Suitable gelling agents for oils (such as mineral oil) include, but are not limited to, hydrogenated butylene/ethylene/styrene copolymer and hydrogenated ethylene/propylene/styrene copolymer. Such gels typically contain between about 0.1% and 5%, by weight, of such gelling agents.

The compositions of the present invention can also be formulated into a solid formulation (e.g., a wax-based stick, soap bar composition, powder, or a wipe containing powder).

The compositions may contain, in addition to the above components, a wide variety of additional oil-soluble materials and/or water-soluble materials conventionally used in compositions for use on skin and hair, at their art-established levels.

Various other materials may also be present in the composition, as known in the art. These include humectants, pH adjusters, chelating agents (e.g., EDTA), fragrances, dyes, and preservatives (e.g., parabens).

The composition and formulations and products containing such compositions of the present invention may be prepared using methodology that is well known by an artisan of ordinary skill.

In one embodiment, the topical composition comprises an emulsion comprising at least two phases selected from an aqueous phase, oil phase, and non-ionic lipid phase.

The aqueous phase contains water.

The aqueous phase may also contain structuring agents such as carbomers or other thickeners, for example, xanthan gum, carageenan gum, polyacrylate-13; polyisobutene; polysorbate-20; polyacrylate-13/polyisobutylene/polysorbate-20 blends, and Stearalkonium Hectorite, including mixtures thereof.

Preferably, the composition comprises a thickener and the thickener is hydroxyethyl acrylate/sodium acryloyldimethyl taurate copolymer.

The oil phase contains at least one cosmetically-acceptable oil.

As used herein, the term "oil" means a hydrophobic material that can aid in balancing the intermolecular forces to form micelle aggregates or to limit their sizes. Oils also serve as emollient ingredients to benefit product spreadibility, skin feel and delivery of hydrophobic active ingredients such as but not limited to, Vitamins D, E, K and A, and sunscreen filters.

Oils that are useful in the composition include a variety of hydrocarbon-based oils, silicones, fatty acid derivatives, glycerides, vegetable oils, vegetable oil derivatives, alkyl esters, wax esters, beeswax derivatives, sterols, and phospholipids and combinations thereof ranging from approximately 20% to 50%, based on the total weight of the composition.

Suitable hydrocarbon oils include petrolatum, mineral oil, micro-crystalline waxes, squalene and combinations thereof.

Silicone oils include dimethicone, dimethiconol, phenyl dimethicone and cyclic polysiloxanes and combinations thereof. Silicone oils having viscosities from about 0.5 to about 100,000 centistokes at 25° C. may also be useful in the composition.

Glycerides include castor oil, sunflower seed oil, coconut oil and derivatives, vegetable oils and derivatives, palm oil, jojoba oil, Shea butter, lanolin and combinations thereof.

Alkyl ester oils include, but are not limited to, isopropyl esters of fatty acids and esters of long chain fatty acids. More preferably, the following alkyl esters are useful: isopropyl palmitate, isopropyl myristate, myristyl myristate, isohexyl palmitate, decyl oleate, isononyl isononanoate and combinations thereof.

The non-ionic lipid phase comprises one or more non-ionic lipids, such as glyceryl monoesters having a fatty acid chain containing from about 3 to about 50 carbon atoms, and preferably from about 10 to about 18 carbon atoms; glyceryl diesters having a fatty acid chain containing from about 5 carbon atoms to about 25 carbon atoms, and preferably from about 10 carbon atoms to about 18 carbon atoms; alkoxylated alcohols; alkoxylated alkyl phenols; alkoxylated acids; alkoxylated amides; alkoxylated sugar derivatives; alkoxylated derivatives of natural oils or waxes; polyoxyethylene polyoxypropylene block copolymers; polyoxyethylene ether fatty acids having a fatty acid chain containing from about 10 carbon atoms to about 18 carbon atoms; steroids; fatty acid esters of alcohols where the fatty acid is straight or branched chain having from about 10 carbon atoms to about 20 carbon atoms and the alcohol is straight or branched chain having 1 to 10 carbon atoms; and mixtures thereof, wherein the alkoxylated lipids are alkoxylated with ethylene oxide or propylene oxide, with ethylene oxide being preferred.

Examples of suitable glyceryl monoesters include, but are not limited to, glyceryl caprate, glyceryl caprylate, glyceryl cocate, glyceryl erucate, glyceryl hydroxysterate, glyceryl isostearate, glyceryl lanolate, glyceryl laurate, glyceryl linolate, glyceryl myristate, glyceryl oleate, glyceryl PABA, glyceryl palmitate, glyceryl ricinoleate, glyceryl stearate, glyceryl thiglycolate, and mixtures thereof, with glyceryl laurate and glyceryl myristate being preferred.

Examples of suitable glyceryl diesters include, but are not limited to, glyceryl dilaurate, glyceryl dioleate, glyceryl dimyristate, glyceryl disterate, glyceryl sesuioleate, glyceryl stearate lactate, and mixtures thereof, with glyceryl dilaurate and glyceryl dimyristate being preferred.

Examples of suitable polyoxyethylene fatty ethers include, but are not limited to, polyoxyethylene cetyl/stearyl ether, polyoxyethylene cholesterol ether, polyoxyethylene laurate or dilaurate, polyoxyethylene stearate or distearate, polyoxyethylene lauryl or stearyl ether, and mixtures thereof, wherein the polyoxyethylene head group ranges from about 2 to about 100 groups. Preferred polyoxyethylene fatty ethers include polyoxyethylene stearyl ether, polyoxyethylene myristyl ether, and polyoxyethylene lauryl ether having from about 3 to about 10 oxyethylene units.

Examples of suitable steroids include, but are not limited to, cholesterol, betasitosterol, bisabolol, and mixtures thereof.

Examples of suitable fatty acid esters of alcohols include isopropyl myristate, aliphati-isopropyl n-butyrate, isopropyl n-hexanoate, isopropyl n-decanoate, isoproppyl palmitate, octyidodecyl myristate.

Exemplary alkoxylated alcohols useful as the nonionic lipid in the compositions of the invention have the structure shown in following formula:

R₅--(OCH₂CH₂)y--OH

wherein R₅ is a branched or unbranched alkyl group having from about 6 to about 22 carbon atoms and y is between about 4 and about 100, and preferably, between about 10 and about 100. A preferred alkoxylated alcohol is the species wherein R₅ is a lauryl group and y has an average value of 23, which is known as laureth 23 and is available from ICI Americas, Inc. of Wilmington, Del. under the tradename "BRIJ 35."

Another exemplary alkoxylated alcohol is an ethoxylated derivative of lanolin alcohol. Lanolin alcohol is a mixture of organic alcohols obtained from the hydrolysis of lanolin. An example of an ethoxylated derivative of lanolin alcohol is laneth-10, which is the polyethylene glycol ether of lanolin alcohol with an average ethoxylation value of 10.

Another exemplary alkoxylated alcohol is polyoxypropylene polyoxyethylene alkyl ether, for example PPG-12-Buteth-16. This material is available from Amerchol Corp. of Edison, N.J. under the tradename, "UCON Fluid 50-HB-660."

Another type of non-ionic lipids includes alkoxylated alkyl phenols, for example nonoxynol-14"and is available under the tradename, "MAKON 14" from the Stepan Company of Northfield, III.

Another type of non-ionic lipids is alkoxylated acids, which are esters of an acid, most usually a fatty acid, with a polyalkylene glycol, for example PEG-8 laurate.

Another type of non-ionic lipids includes alkoxylated amides, for example PEG-6 cocoamide.

Another type of non-ionic lipids includes the alkoxylated sugar derivatives, for instance polysorbate 20, a mixture of laurate esters of sorbitol and sorbitol anhydrides, consisting predominately of the monoester, condensed with about 20 moles of ethylene oxide. This material is available under the tradename "TWEEN 20" from ICI Americas of Wilmington, Del.

Another example of an alkoxylated sugar derivative useful in the compositions of the invention is PEG-20 methylglucose sesquistearate, which is the polyethyleneglycol ether of the sesquiester of methyl glucose and stearic acid, contains an average of 20 moles of ethylene oxide, and is available under the tradename, "GLUCAMATE SSE-20" from the Amerchol Corp. of Edison, N.J.

Another type of non-ionic lipids includes the alkoxylated derivatives of natural oils and waxes. Examples of this class of material include PEG-40 lanolin, PEG-40 castor oil and PEG-40 hydrogenated castor oil.

Another type of non-ionic lipids includes polyoxyethylene polyoxypropylene block copolymers, for example Poloxamer 101 and Poloxamer 182.

Preferred nonionic lipids include polyoxyethylene fatty ethers, glyceryl diesters, and mixtures thereof. More preferred nonionic lipids include polyoxyethylene stearyl ether, polyoxyethylene myristyl ether, and polyoxyethylene lauryl ether, glyceryl dilaurate, glyceryl dimystate, glyceryl distearate, and mixtures thereof, whereby each ether has from about 5 to about 10 oxyethylene units.

In an embodiment wherein the reduction of skin irritation is a concern, it is preferable to use a nonionic lipid having a greater amount of carbon atoms on the hydrophilic head group moiety, or in the alternative, a nonionic lipid having a greater amount of carbon atoms on the hydrophobic fatty acid chain moiety. The former can be achieved by increasing the amount of carbon atoms on the head group of, for example, a polyoxyethylene-10-stearyl ether from about 10 carbon atoms to from about 15 to 20 carbon atoms. The latter can be achieved by increasing the amount of carbon atoms on the 12 carbon fatty acid tail of, for example, glyceryl diesters to from about 14 carbons to about 16 carbons.

The composition of the present invention may include, based upon the total weight of the composition, from about 1 percent to about 10 percent, and preferably from about 3 percent to about 7 percent of the nonionic lipid.

In a preferred embodiment, the non-ionic lipid phase comprises water, glyceride dilaurate, steareth-10, and glycerin.

### pH

In one embodiment, the topical composition has a low pH. For example, the pH may be less than about 4 or less than about 3.3. The composition is not required to have a low pH, however.

The topical composition may comprise a buffering agent such as lactic acid, citric acid, malic acid, tartaric acid, gluconic acid, or gluconolactone. Preferably the buffering agent is lactic acid.

Typically, the composition contains about 3 to about 12, or about 4 to about 8, weight percent of buffering agent.

### Methods of Treating Skin

According to the invention, signs of skin aging may be treated by topically applying a topical composition comprising an N-acyl dipeptide derivative as described herein and up to 10 weight percent of glycolic acid to skin in need of treatment for at least one sign of skin aging.

The penetration of an N-acyl dipeptide derivative into skin may be increased by topically administering it in a composition that also comprises up to 10 weight percent of glycolic acid.

In one embodiment, a composition according to the invention comprising an N-acyl dipeptide derivative and glycolic acid in an amount of up to 10 weight percent of the composition provides at least about a two-fold, preferably about a three-fold, or up to a seven-fold, increase in the cumulative delivery of the N-acyl dipeptide derivative into skin compared with a composition containing either no glycolic acid or greater than 10 weight percent glycolic acid but otherwise the same.

The following non-limiting examples further illustrate the invention. The following Skin Penetration Method and High-Performance Liquid Chromatography-Mass Spectrometry (HPLC-MS) Method were employed.

### Skin Penetration Method

*In vitro* skin permeation experiments were performed under the guidelines outlined by the Organization for Economic Cooperation and Development (OECD).

Split-thickness human cadaver skin specimens were dermatomed to a nominal thickness of 300 µm and were stored at -80°C in Roswell Park Memorial Institute (RPMI)-1640 solution preserved with oxacillin sodium and gentamicin. One entire package (ca. ¼ sq. ft.) of skin, obtained from a single donor, was used in each skin penetration study to accommodate ca. 50-60 skin samples per study. When ready for use, the skin was rapidly thawed and rinsed in deionized water prior to being cut into ca. 2.25 cm² pieces that were mounted in static, vertical glass Franz diffusion cells. The non-occluded donor compartment was comprised of a low glass cap extending 12 mm above an exposed surface area of 0.79 cm². The receptor compartment contained 4.5 mL of Dulbecco's phosphate-buffered (pH 7.4) saline with 0.02% sodium azide to provide a physiologically compatible fluid that retards microbial growth. Each Franz diffusion cell was placed in an aluminum slot of Pierce Reacti-Therm^{™} Heating and Stirring Modules (Rockford, IL) set at human body temperature (37°C) to attain a skin surface temperature of 32°C comparable to *in vivo* conditions. The receptor solution was stirred at approximately 400 rpm with magnetic stir bars to ensure homogeneity of the reservoir contents.

Skin integrity testing was performed using a tritiated water (3H₂O) screening procedure prior to the start of the skin penetration measurements to ensure intact barrier function. The skin samples were equilibrated in the Franz diffusion cells for 2 hours to ensure partial hydration of the stratum corneum to mimic *in vivo* skin conditions as opposed to conducting the skin penetration testing on fully hydrated skin immediately after thawing which would increase skin permeability. A 150 µL dose of 3H₂0 solution (0.4 µCi/mL) was added to each donor compartment and subsequently removed from the skin surface after a 5-minute contact time using a cotton-tipped swab. The receptor compartment contents were collected 30 minutes post-dose and immediately refilled with fresh receptor solution maintained at 37°C. The receptor samples were mixed with 10 mL of Ultima Gold XR liquid scintillation cocktail and counted for 1 minute by a Beckman LS6500 Liquid Scintillation Counter. Skin samples with 3H₂O permeation greater than 2.0 µL/cm² after a 30-minute exposure duration were rejected from use in the skin penetration study and were instead used as untreated controls to provide blank matrices for analytical quantitation or to assess extraction efficiencies of the test dipeptide derivative from the skin samples. The skin samples that passed the skin integrity test were rank ordered according to 3H₂O permeability and each sample was assigned to a treatment group based on a randomized complete block design to account for the inherent variability in skin permeability to enable more robust treatment effect comparisons on dipeptide derivative skin penetration. The Franz diffusion cells were washed out several times to remove residual 3H, equilibrated overnight, and washed out in the morning with receptor solution containing 0.1% Oleth-20 which was used for the rest of the skin penetration study as a solubility enhancer to maintain sink conditions.

Semi-solid test formulations were vortexed immediately prior to use, and throughout the dosing regimen if needed, to thoroughly mix the contents. The skin sample surface was dried prior to dosing by gently dabbing the skin with cotton swabs then the skin samples were dosed with the designated treatments. A minimum of 4 replicates per treatment were tested. For all test formulations, 5 µl of the test formulation was deposited onto the skin with a positive displacement pipette. The formulation was spread evenly over the entire surface area of the exposed tissue (0.79cm₂) using a glass stir rod that was pre-wetted with the test formulation. Care was taken to ensure that the viscous dose remained on the skin surface and not along the walls of the donor chamber. The amount of test formulation added/removed during the spreading procedure was accounted for gravimetrically. The dosing was spaced out according to the time required to complete the terminal receptor compartment sampling at 48 h post-dosing, skin surface wash, tape stripping, and epidermal/dermal skin fractionation to enable uniform collection times for all samples.

The entire receptor fluid contents were collected after 6, 24, and 48 hours following dosing and the receptor compartment was replenished with fresh receptor solution after the 6- and 24-hour collections. Following the 48-hour exposure duration and terminal receptor compartment sampling, the unabsorbed formulation was removed by washing with a dilute and mild surfactant solution comprised of 5% oleth-20 in DI water. A 300 µL aliquot of the wash solution was applied to the skin surface with a positive-displacement pipette, agitated for 30 seconds with a glass stir rod to mimic an *in vivo* wash procedure, and then transferred from the donor compartment into a 1.5 Eppendorf tube using a plastic transfer pipette. The skin was then rinsed twice with 500 µL aliquots of DI water that were stirred on the skin surface for 30 seconds with the glass stir rod and collected in the same tube as the surfactant wash. The donor chamber was removed and rinsed with 1.5 mL of 2:1 methanol:water extraction solvent to collect residual test article concentrations that were unabsorbed after 48 h and this volume was pipetted into a separate 1.5 Eppendorf tube. The skin surface was allowed to air dry for approximately 30 minutes prior to tape-stripping off the top stratum corneum layer to remove the potentially unabsorbed dose that was either not adequately removed from the skin surface wash or to mimic the amount that would be removed *in vivo* via desquamation after 48 hours. To accomplish this, the skin specimens were placed on a glass plate, D100 D-Squame tapes were placed over the 0.79 cm² treated surface area, and uniform pressure was applied for 15 seconds with the D-Squame disc applicator per the manufacturer instructions. The tape was removed with forceps and placed in the Eppendorf tube containing the donor chamber rinse with the sticky side facing inward while ensuring that the tape strip was adequately submerged in the extraction solvent. Forceps were then used to carefully peel the epidermis from the dermis and the separated skin samples were placed into separate 2 mL short vials. 1.5 mL of 2:1 methanol:water extraction solvent was added to each of the vials containing the epidermal and dermal skin samples. Dipeptide derivative concentrations in the skin surface wash, tape strip, and skin samples were extracted by shaking the samples on the orbital shaker at room temperature for 24 hours at 150 rpm. Samples were subsequently filtered through a syringe filter containing a 0.45 µm membrane and collected in 2 mL HPLC vials for analytical quantitation.

For mass balance calculations, the total analyte content in each of the formulations (3 replicates per formulation) was measured by pipetting the 5 µL dose into a one-dram shell vial containing 2 mL extraction solvent (2:1 methanol:water). Samples were shaken on an orbial shaker at room temperature for 48 h at 150 rpm prior and subsequently filtered through a syringe filter containing a 0.45 µm membrane and collected in 2 mL HPLC vials for analytical quantitation.

### High-Performance Liquid Chromatography-Mass Spectrometry (HPLC-MS) Method

High-Performance Liquid Chromatography-Mass Spectrometry (HPLC-MS) was used to analyze the quantity of N-Acetyl-L-valine-L-Alaninamide in the tape strips (T), epidermis (E), dermis (D), wash (W) and receptor (R) collected using the Skin Penetration Method.

2-acetamido-N-(2-amino-2- oxoethyl)-3-methylbutanamide (Aurora Fine Chemicals LLC, Purity > 95%, Batch# A06.430.527_4) was used as an internal standard (IS). An Agilent HPLC/MSD system was used, System ID#: SK_1519. Mobile phases used were A: 0.1% formic acid (FA) (Sigma, reagent grade (>95%), Lot#: SHBJ2924) in water (EMD, HPLC grade, Lot#: 57256), and B: 0.1% FA (Sigma) and 2mM Ammonium Formate (Fluka, HPLC grade, Lot#: BCBQ4532V) in methanol (EMD, HPLC grade, Lot#: 25777). The column was a Zorbax Eclipse XDB-C18 column (3.5 µm, 150 × 4.6 mm ID, S/N: USWA016962) from Agilent, and was used at 40°C. Binary gradient elution was used as shown in Table X. The detector was programed using the parameters outlined in Table Y

**Table X**

| Time (min) | Flow Rate (ml/min) | Mobile Phase A (%) | Mobile Phase B (%) |
|---|---|---|---|
| 0.0 | 0.8 | 90.0 | 10.0 |
| 0.5 | 0.8 | 90.0 | 10.0 |
| 8.0 | 0.8 | 10.0 | 90.0 |
| 9.0 | 0.8 | 10.0 | 90.0 |
| 9.1 | 0.8 | 90.0 | 10.0 |
| 14.0 | 0.8 | 90.0 | 10.0 |

**Table Y**

| **MS Parameters** | **Values** |
|---|---|
| Positive ions monitored (amu) | N-Acetyl-L-valine-L-Alaninamide: 229.8 |
| | IS: 216.0 |
| Fragment | 70 |
| Drying gas flow (I/min) | 12 |
| Nebulizer pressure (psig) | 31 |
| Drying gas Temperature | 20 |
| Capillary voltage (IS) | 5,000 |

A stock standard solution (1.0 mg/mL) of N-Acetyl-L-valine-L-Alaninamide was prepared as follows. Approximately 50g of an N-Acetyl-L-valine-L-Alaninamide reference standard was placed into a 50mL volumetric flask, and 30mL of MeOH was added. The mixture was sonicated for 2mins, diluted to mark with MeOH, and mixed well. The stock standard solution was followed with serial dilutions in the diluent (MeOH: Water 2:1, v/v) to make the working standards (W-STDs) at final concentrations of 0.1, 0.2, 0.5, 2.0, and 5.0 µg/mL and working quality control standards (W-QCs) at final concentrations of 0.3, 1.0 and 4.0 µg/mL. Internal standard stock solution (1.0 mg/mL) was prepared in MeOH followed by dilution in diluent to make a working internal solution (W-IS) at concentration of 100 ng/mL.

A calibration standard was prepared as follows. 180 µL of diluent was added to a 2.0 mL HPLC vial. Next, 20 µl of W-STD solution or diluent (for blank) was added, then 800 µl of internal standard working solution at 100 ng/mL or diluent (for double blank) was added and vortexed for 1 minute. Quality control (QC) samples were prepared as follows. 180 µL of epidermis or dermis or wash or receptor blank matrix was added to a 2.0 mL HPLC vial, and 20 µL of W-QC solution was added. 800 µL of internal standard working solution at 100 ng/mL was added to the HPLC vial and vortexed for 1 minute.

Test samples were prepared as follows. 200 µL of sample was added to a 2.0 ml HPLC vial. 800 µL of internal standard working solution at 100 ng/mL was added and vortexed for 1 minute. The sample solution was diluted with MeOH:H2O (2:1, v/v) as needed. Calibration standards, QC samples, and test samples were subjected to HPLC-MS analysis using the conditions described above.

### Example 1

A series of test compositions containing N-Ac-Val-Ala-NH₂ were tested for penetration into human skin samples using the Skin Penetration Method and HPLC-MS Method. The compositions contained the ingredients shown in Tables 1, 2 and 3. The amounts of ingredients are reported in percent by weight based on the total weight of the composition. Compositions A-D and F-G were comparative. Composition 1 and Composition E were according to the invention.

N-Ac-Val-Ala-NH₂ was obtained from Nanjing Pharmatechs Co., Ltd. It was a powder.

Glycolic acid was in the form of a 70 wt% solution in water (GLYPURE, commercially available from DuPont).

**TABLE 1**

| **INCI Name** | **Composition A** | **Composition B** | **Composition C** | **Composition D** | **Composition E** | **Composition 1** |
|---|---|---|---|---|---|---|
| Purified Water USP | 62.04 | 62.54 | 62.94 | 57.54 | 51.54 | 58.54 |
| Magnesium Aluminum Silicate | 0.15 | 0.15 | 0.15 | 0.15 | 0.15 | 0.15 |
| Disodium EDTA | 0.03 | 0.03 | 0.03 | 0.03 | 0.03 | 0.03 |
| Glycolic Acid | 0 | 0 | 0 | 0 | 0 | 4.00 |
| N-Acetyl Valyl Alanylamide | 1.00 | 0.50 | 0.10 | 0.50 | 0.50 | 0.50 |
| Propylene Glycol | 16.00 | 16.00 | 16.00 | 16.00 | 16.00 | 16.00 |
| Xanthan Gum | 0.08 | 0.08 | 0.08 | 0.08 | 0.08 | 0.08 |
| Sorbitan Stearate | 1.95 | 1.95 | 1.95 | 1.95 | 1.95 | 1.95 |
| PEG-40 Stearate | 1.95 | 1.95 | 1.95 | 1.95 | 1.95 | 1.95 |
| White Petrolatum USP/NF | 4.80 | 4.80 | 4.80 | 4.80 | 4.80 | 4.80 |
| Stearyl Alcohol | 2.40 | 2.40 | 2.40 | 2.40 | 2.40 | 2.40 |
| Cetyl Alcohol | 0.60 | 0.60 | 0.60 | 0.60 | 0.60 | 0.60 |
| Dimethicone | 0.60 | 0.60 | 0.60 | 0.60 | 0.60 | 0.60 |
| White Beeswax SP-422P | 2.40 | 2.40 | 2.40 | 2.40 | 2.40 | 2.40 |
| Polysorbate 80 | 0 | 0 | 0 | 5.00 | 0 | 0 |
| Mineral Oil | 4.80 | 4.80 | 4.80 | 4.80 | 4.80 | 4.80 |
| Caprylyl Glycol/Chlorophenesin/Phen oxyethanol | 1.20 | 1.20 | 1.20 | 1.20 | 1.20 | 1.20 |
| Purified Water USP | 0 | 0 | 0 | 0 | 9.00 | 0 |
| Glyceryl Dilaurate | 0 | 0 | 0 | 0 | 1.00 | 0 |
| Steareth-10 | 0 | 0 | 0 | 0 | 0.50 | 0 |
| Glycerin | 0 | 0 | 0 | 0 | 0.50 | 0 |
| Total | 100.00 | 100.00 | 100.00 | 100.00 | 100.00 | 100.00 |
| pH | 4.87 | 5.33 | 4.92 | 5.14 | 4.80 | 3.80 |

**TABLE 2**

| **Composition F** | |
|---|---|
| INCI Name | Weight Percent |
| Dimethicone, Cyclopentasiloxane, Polysilicone-11, Nylon-12, Silica, PEG-10 Dimethicone, Polysorbate 40, Isohexadecane, Ammonium Polyacrylodimetyhl Taurate | 39.50 |
| Hydroxyethyl Acrylate/Sodium Acryloyl Dimethyl Taurate Copolymer | 0.40 |
| Vitamin E Acetate | 1.00 |
| Xanthan Gum | 0.20 |
| PEG-10 Dimethicone | 0.80 |
| Phenoxyethanol, Chlorphenesin, Caprylyl Glycol | 1.00 |
| Butyloctyl Salicylate | 5.00 |
| BHT | 0.98 |
| Purified Water | 45.25 |
| N-Acetyl Valyl Alanylamide | 0.50 |
| Glycerin | 5.00 |
| Citric Acid | 0.27 |
| Disodium EDTA | 0.10 |
| Total | 100.00 |
| pH | 2.90 |

**TABLE 3**

| **Composition G** | |
|---|---|
| **INCI Name** | **Weight Percent** |
| Purified Water | 73.25 |
| N-Acetyl Valyl Alanylamide | 0.50 |
| Propylene Glycol | 5.00 |
| Hydroxyethylcellulose | 0.15 |
| Xanthan Gum | 0.30 |
| Disodium EDTA | 0.10 |
| Dimethicone | 2.00 |
| Stearic Acid | 2.75 |
| Glyceryl Stearate/PEG-100 Stearate | 4.00 |
| Dicapryl ether | 4.00 |
| Isopropyl Palmitate | 2.75 |
| Cetyl Alcohol | 4.00 |
| Phenoxyethanol, Caprylyl Glycol, Chlorphenesin | 1.20 |
| Total | 100 |
| pH | 4.22 |

The results are shown in Table 4.

**TABLE 4**

| Test Composition | Amounts (ng/cm²) collected after 48 hours, mean ± standard deviation, n = 4/treatment | | | | |
|---|---|---|---|---|---|
| | Cumulative permeated through skin^{a} | Cumulative penetrated into skin^{b} | Total bioavailable in skin^{c} | Unabsorbed^{d} | Total recovered |
| Composition A | 0 ± 0 | 257 ± 190 | 257 ± 190 | 67771 ± 23934 | 68028 ± 24053 |
| Composition B | 163 ± 190 | 496 ± 393 | 658 ± 386 | 28746 ± 17471 | 29404 ± 17824 |
| Composition C | 75 ± 125 | 601 ± 240 | 677 ± 220 | 5864 ± 2265 | 6540 ± 2440 |
| Composition D | 130 ± 205 | 232 ± 99 | 362 ± 169 | 42453 ± 26119 | 42815 ± 26057 |
| Composition 1 | 576 ± 424 | 4135 ± 2472 | 4711 ± 2057 | 23627 ± 5711 | 28338 ± 7580 |
| Composition E | 2019 ± 2657 | 635 ± 550 | 2654 ± 3203 | 20995 ± 2694 | 23649 ± 4363 |
| Composition F | 13 ± 26 | 640 ± 140 | 653 ± 137 | 29335 ± 9880 | 29988 ± 9963 |
| Composition G | 136 ± 154 | 564 ± 223 | 701 ± 293 | 24885 ± 1363 | 25585 ± 1546 |
| ^{a} Sum of amounts collected in the receptor compartment at 6, 24, and 48 hours post-dosing | | | | | |
| ^{b} Sum of amounts extracted from the epidermis and dermis | | | | | |
| ^{c} Sum of cumulative amounts permeated through skin (column 1) and cumulative amounts penetrated into skin (column 2) | | | | | |
| ^{d} Sum of amounts removed from the skin surface wash and extracted from the tape-strip | | | | | |

The above results show Composition 1 containing a combination of N-Acetyl Valyl Alanylamide and glycolic acid provided the greatest skin penetration of N-Acetyl Valyl Alanylamide of all the compositions tested. Significantly greater (p < 0.01) cumulative amounts of dipeptide derivative were delivered into skin by Composition 1 compared to all the other compositions, and it provided the highest overall bioavailable amount of all the compositions with significantly (p < 0.05) higher bioavailable amounts compared to all compositions except for Composition E. For example, Composition 1 delivered about seven times the total bioavailable amount of dipeptide derivative into skin compared with that provided by Composition B.

It may be noted that Composition E delivered four times the total bioavailable amount of dipeptide derivative into skin compared to Composition B, indicating that the presence of glyceryl dilaurate, steareth-10, and glycerin in the formulation also increases the delivery of a dipeptide derivative into the skin.

In contrast, increasing the dose of N-Acetyl Valyl Alanylamide alone did not increase its skin penetration.

### Example 2

A series of test compositions containing 0.5 wt% of N-Ac-Val-Ala-NH₂ and 0, 1, 4, 8, or 11 wt % of glycolic acid were tested for penetration into human skin samples using the Skin Penetration Method and HPLC-MS Method. The compositions contained the ingredients shown in Table 5. The amounts of ingredients are reported in percent by weight based on the total weight of the composition. Compositions H-L were comparative. Compositions 2-4 were according to the invention.

The N-Ac-Val-Ala-NH₂ and glycolic acid were obtained as described in Example 1.

**TABLE 5**

| **INCI Name** | **Composition H** | **Composition 2** | **Composition 3** | **Composition 4** | **Composition I** | **Composition J** | **Composition K** | **Composition L** |
|---|---|---|---|---|---|---|---|---|
| Purified Water USP | 62.54 | 61.54 | 58.54 | 54.53 | 51.53 | 62.44 | 58.54 | 58.54 |
| Magnesium Aluminum Silicate | 0.15 | 0.15 | 0.15 | 0.15 | 0.15 | 0.15 | 0.15 | 0.15 |
| Disodium EDTA | 0.03 | 0.03 | 0.03 | 0.03 | 0.03 | 0.03 | 0.03 | 0.03 |
| Gluconolactone | 0 | 0 | 0 | 0 | 0 | 0 | 4.00 | 0 |
| Glycolic Acid (70% Active) | 0 | 1.00 | 4.00 | 8.01 | 11.01 | 0 | 0 | 0 |
| Mandelic Acid | 0 | 0 | 0 | 0 | 0 | 0 | 0 | 4.00 |
| N-Acetyl Valyl Alanylamide | 0.50 | 0.50 | 0.50 | 0.50 | 0.50 | 0.50 | 0.50 | 0.50 |
| Propylene Glycol | 16.00 | 16.00 | 16.00 | 16.00 | 16.00 | 16.00 | 16.00 | 16.00 |
| Xanthan Gum | 0.08 | 0.08 | 0.08 | 0.08 | 0.08 | 0.08 | 0.08 | 0.08 |
| Sorbitan Stearate | 1.95 | 1.95 | 1.95 | 1.95 | 1.95 | 1.95 | 1.95 | 1.95 |
| PEG-40 Stearate | 1.95 | 1.95 | 1.95 | 1.95 | 1.95 | 1.95 | 1.95 | 1.95 |
| White Petrolatum USP/NF | 4.80 | 4.80 | 4.80 | 4.80 | 4.80 | 4.80 | 4.80 | 4.80 |
| Stearyl Alcohol | 2.40 | 2.40 | 2.40 | 2.40 | 2.40 | 2.40 | 2.40 | 2.40 |
| Cetyl Alcohol | 0.60 | 0.60 | 0.60 | 0.60 | 0.60 | 0.60 | 0.60 | 0.60 |
| Dimethicone | 0.60 | 0.60 | 0.60 | 0.60 | 0.60 | 0.60 | 0.60 | 0.60 |
| White Beeswax SP-422P | 2.40 | 2.40 | 2.40 | 2.40 | 2.40 | 2.40 | 2.40 | 2.40 |
| Mineral Oil | 4.80 | 4.80 | 4.80 | 4.80 | 4.80 | 4.80 | 4.80 | 4.80 |
| Caprylyl Glycol / Chlorphenesin / Phenoxyethanol | 1.20 | 1.20 | 1.20 | 1.20 | 1.20 | 1.20 | 1.20 | 1.20 |
| Hydrochloric Acid (10% Solution) | 0 | 0 | 0 | 0 | 0 | 0.10 | 0 | 0 |
| Total | 100.00 | 100.00 | 100.00 | 100.00 | 100.00 | 100.00 | 100.00 | 100.00 |
| pH | 5.33 | 3.84 | 3.80 | 3.80 | 3.80 | 3.83 | 3.81 | 3.80 |

The results are shown in Table 6.

**TABLE 6**

| Test Composition | Amounts (ng/cm²) collected after 48 hours, mean ± standard deviation, n = 4/treatment | | | | |
|---|---|---|---|---|---|
| | Cumulative permeated through skin^{a} | Cumulative penetrated into skin^{b} | Total bioavailabl e in skin^{c} | Unabsorbed^{d} | Total recovered |
| Composition H | 50 ± 99 | 1173 ± 1163 | 1222 ± 1261 | 29496 ± 5461 | 30768 ± 4876 |
| Composition 2 | 0 ± 0 | 3891 ± 1269 | 3891 ± 1269 | 23265 ± 5986 | 27156 ± 5734 |
| Composition 3 | 0 ± 0 | 4183 ± 810 | 4183 ± 810 | 29023 ± 7708 | 33206 ± 7753 |
| Composition 4 | 140 ± 192 | 2982 ± 1087 | 3122 ± 1058 | 47535 ± 8320 | 50798 ± 7535 |
| Composition I | 9 ± 17 | 1728 ± 1021 | 1736 ± 1011 | 33296 ± 6725 | 35041 ± 7338 |
| Composition J | 0 ± 0 | 1067 ± 298 | 1067 ± 298 | 38550 ± 6342 | 39614 ± 6252 |
| Composition K | 425 ± 824 | 1611 ± 689 | 2037 ± 1442 | 57813 ± 13010 | 60275 ± 12290 |
| Composition L | 10 ± 20 | 1824 ± 1381 | 1834 ± 1374 | 32142 ± 3665 | 33986 ± 4175 |
| ^{a} Sum of amounts collected in the receptor compartment at 6, 24, and 48 hours post-dosing | | | | | |
| ^{b} Sum of amounts extracted from the epidermis and dermis | | | | | |
| ^{c} Sum of cumulative amounts permeated through skin (column 1) and cumulative amounts penetrated into skin (column 2) | | | | | |
| ^{d} Sum of amounts removed from the skin surface wash and extracted from the tape-strip | | | | | |

These results show Compositions 2, 3 and 4 containing 1%, 4% and 8% glycolic acid, respectively, delivered the most dipeptide derivative into the skin. These compositions each delivered significantly (p < 0.05) more cumulative amounts of the dipeptide derivative into the skin than Composition **H,** the same base formula containing 0% glycolic acid, providing significantly higher bioavailable concentrations of the dipeptide derivative in skin. For example, Compositions 2-4 delivered about three times the total bioavailable amount of dipeptide derivative into skin compared with that provided by Composition H.

Surprisingly, increasing the glycolic acid concentration to 11 wt% (Composition I) decreased delivery of the dipeptide derivative into skin compared to the 1%, 4% and 8% glycolic acid compositions, and significantly (p < 0.05) decreased delivery of the dipeptide derivative into skin compared to the 1% and 4% glycolic acid compositions. For example, Compositions I delivered about two to three times lower total bioavailable amounts of dipeptide derivative into skin compared with that provided by Compositions 2-3.

Moreover, Composition 3 containing 4 wt% glycolic acid provided two times the bioavailable amount of dipeptide derivative in skin compared to Composition K and Composition L containing 4 wt% of gluconolactone and mandelic acid, respectively.

Finally, mere adjustment of pH in the base formula (Composition H) to 3.8, the pH of glycolic acid containing compositions, did not impact the level of dipeptide derivative delivered to the skin, as shown by the results for Composition J.

## Claims

1. A topical composition comprising an N-acyl dipeptide derivative having the formula:
R₁-Val-Ala-R₂
or an isomer or salt thereof, wherein Val is valine, Ala is alanine, R₁ is an acyl radical having up to 19 carbon atoms; R₂ is OR₃, NHR₄, or NHNHR₅; R₃ is H, an alkyl, aralkyl, or aryl radical having up to 19 carbon atoms; and R₄ and R₅ are each independently H, OH, an alkyl, aralkyl, aryl, or acyl radical having up to 19 carbon atoms, and glycolic acid, wherein the composition comprises up to 10 weight percent of glycolic acid.

2. The topical composition of claim 1 comprising 1 to 8 weight percent of glycolic acid.

3. The topical composition of claim 1 or claim 2, wherein the dipeptide derivative is selected from the group consisting of N-Ac-Val-Ala-NH₂, N-Ac-Val-Ala-OH, N-Ac-Val-Ala-NHOH, N-Pr-Val-Ala-NH₂, and N-Pr-Val-Ala-OH, wherein Ac is acetyl and Pr is propanoyl.

4. The topical composition of any one of claims 1 to 3, wherein the N-acyl dipeptide derivative is N-Ac-Val-Ala-NH₂ wherein Ac is acetyl.

5. The topical composition of any one of claims 1 to 4, comprising 0.01 to 2 weight percent of N-Ac-Val-Ala-NH₂ wherein Ac is acetyl.

6. The topical composition of any one of claims 1 to 5, further comprising a cosmetically acceptable topical carrier.

7. The topical composition of claim 6, wherein the carrier comprises one or more of glyceryl dilaurate, steareth-10, and glycerin.

8. The topical composition of claim 6 or claim 7, wherein the carrier comprises water.

9. The topical composition of any one of claims 6 to 8, wherein the carrier comprises water, glyceryl dilaurate, steareth-10 and glycerin.

10. The topical composition of any preceding claim, comprising 0.01 to 2 weight percent of N-Ac-Val-Ala-NH₂ wherein Ac is acetyl, 1 to 8 weight percent of glycolic acid, and a cosmetically acceptable topical carrier.

11. A topical composition comprising an N-acyl dipeptide derivative having the formula:
R₁-Val-Ala-R₂
or an isomer or salt thereof, wherein Val is valine, Ala is alanine, R₁ is an acyl radical having up to 19 carbon atoms; R₂ is OR₃, NHR₄, or NHNHR₅; R₃ is H, an alkyl, aralkyl, or aryl radical having up to 19 carbon atoms; and R₄ and R₅ are each independently H, OH, an alkyl, aralkyl, aryl, or acyl radical having up to 19 carbon atoms, and a cosmetically acceptable topical carrier comprising glyceryl dilaurate, steareth-10, and glycerin.

12. A non-therapeutic method of treating signs of skin aging, comprising topically applying to skin in need of treatment for at least one sign of skin aging a topical composition comprising an N-acyl dipeptide derivative having the formula:
R₁-Val-Ala-R₂
or an isomer or salt thereof, wherein Val is valine, Ala is alanine, R₁ is an acyl radical having up to 19 carbon atoms; R₂ is OR₃, NHR₄, or NHNHR₅; R₃ is H, an alkyl, aralkyl, or aryl radical having up to 19 carbon atoms; and R₄ and R₅ are each independently H, OH, an alkyl, aralkyl, aryl, or acyl radical having up to 19 carbon atoms, and glycolic acid, wherein the composition comprises up to 10 weight percent of glycolic acid.

13. The non-therapeutic method of claim 12, wherein the sign of skin aging is selected from the group consisting of lines, fine lines, wrinkles, loss of elasticity, uneven skin, blotchiness, diminished skin thickness, and abnormal or diminished synthesis of collagen, glycosaminoglycans, proteoglycans, or elastin.

## Patentansprüche

1. Topische Zusammensetzung, umfassend ein N-Acyldipeptidderivat mit der Formel:
R₁-Val-Ala-R₂
oder ein Isomer oder Salz davon, wobei Val für Valin steht, Ala für Alanin steht, R₁ für einen Acylrest mit bis zu 19 Kohlenstoffatomen steht; R₂ für OR₃, NHR₄ oder NHNHR₅ steht; R₃ für H oder einen Alkyl-, Aralkyl- oder Arylrest mit bis zu 19 Kohlenstoffatomen steht und R₄ und R₅ jeweils unabhängig für H, OH oder einen Alkyl-, Aralkyl-, Aryl- oder Acylrest mit bis zu 19 Kohlenstoffatomen stehen, und Glykolsäure, wobei die Zusammensetzung bis zu 10 Gewichtsprozent Glykolsäure umfasst.

2. Topische Zusammensetzung nach Anspruch 1, umfassend 1 bis 8 Gewichtsprozent Glykolsäure.

3. Topische Zusammensetzung nach Anspruch 1 oder Anspruch 2, wobei das Dipeptidderivat aus der Gruppe bestehend aus N-Ac-Val-Ala-NH₂, N-Ac-Val-Ala-OH, N-Ac-Val-Ala-NHOH, N-Pr-Val-Ala-NH₂ und N-Pr-Val-Ala-OH ausgewählt ist, wobei Ac für Acetyl steht und Pr für Propanoyl steht.

4. Topische Zusammensetzung nach einem der Ansprüche 1 bis 3, wobei es sich bei dem N-Acyldipeptidderivat um N-Ac-Val-Ala-NH₂ handelt, wobei Ac für Acetyl steht.

5. Topische Zusammensetzung nach einem der Ansprüche 1 bis 4, umfassend 0,01 bis 2 Gewichtsprozent N-Ac-Val-Ala-NH₂, wobei Ac für Acetyl steht.

6. Topische Zusammensetzung nach einem der Ansprüche 1 bis 5, ferner umfassend einen kosmetisch unbedenklichen topischen Träger.

7. Topische Zusammensetzung nach Anspruch 6, wobei der Träger eines oder mehrere von Glyceryldilaurat, Steareth-10 und Glycerin umfasst.

8. Topische Zusammensetzung nach Anspruch 6 oder Anspruch 7, wobei der Träger Wasser umfasst.

9. Topische Zusammensetzung nach einem der Ansprüche 6 bis 8, wobei der Träger Wasser, Glyceryldilaurat, Steareth-10 und Glycerin umfasst.

10. Topische Zusammensetzung nach einem der vorhergehenden Ansprüche, umfassend 0,01 bis 2 Gew.-% N-Ac-Val-Ala-NH₂, wobei Ac für Acetyl steht, 1 bis 8 Gewichtsprozent Glykolsäure und einen kosmetisch unbedenklichen topischen Träger.

11. Topische Zusammensetzung, umfassend ein N-Acyldipeptidderivat mit der Formel:
R₁-Val-Ala-R₂
oder ein Isomer oder Salz davon, wobei Val für Valin steht, Ala für Alanin steht, R₁ für einen Acylrest mit bis zu 19 Kohlenstoffatomen steht; R₂ für OR₃, NHR₄ oder NHNHR₅ steht; R₃ für H oder einen Alkyl-, Aralkyl- oder Arylrest mit bis zu 19 Kohlenstoffatomen steht und R₄ und R₅ jeweils unabhängig für H, OH oder einen Alkyl-, Aralkyl-, Aryl- oder Acylrest mit bis zu 19 Kohlenstoffatomen stehen, und einen kosmetisch unbedenklichen topischen Träger, der Glyceryldilaurat, Steareth-10 und Glycerin umfasst.

12. Nichttherapeutisches Verfahren zur Behandlung von Anzeichen von Hautalterung, umfassend das topische Aufbringen einer topischen Zusammensetzung, die ein N-Acyldipeptidderivat mit der folgenden Formel umfasst, auf Haut, die einer Behandlung für mindestens ein Anzeichen von Hautalterung bedarf:
R₁-Val-Ala-R₂
oder ein Isomer oder Salz davon, wobei Val für Valin steht, Ala für Alanin steht, R₁ für einen Acylrest mit bis zu 19 Kohlenstoffatomen steht; R₂ für OR₃, NHR₄ oder NHNHR₅ steht; R₃ für H oder einen Alkyl-, Aralkyl- oder Arylrest mit bis zu 19 Kohlenstoffatomen steht und R₄ und R₅ jeweils unabhängig für H, OH oder einen Alkyl-, Aralkyl-, Aryl- oder Acylrest mit bis zu 19 Kohlenstoffatomen stehen, und Glykolsäure, wobei die Zusammensetzung bis zu 10 Gewichtsprozent Glykolsäure umfasst.

13. Nichttherapeutisches Verfahren nach Anspruch 12, wobei das Zeichen der Hautalterung aus der Gruppe bestehend aus Linien, feinen Linien, Falten, Elastizitätsverlust, unebener Haut, Fleckigkeit, verminderter Hautdicke und abnormaler oder verminderter Synthese von Kollagen, Glykosaminoglykanen, Proteoglykanen oder Elastin ausgewählt ist.

## Revendications

1. Composition topique comprenant un dérivé N-acyldipeptide de formule :
R₁-Val-Ala-R₂
ou un isomère ou un sel de celui-ci, dans laquelle Val est la valine, Ala est l'alanine, R₁ est un radical acyle ayant jusqu'à 19 atomes de carbone ; R₂ est OR₃, NHR₄, ou NHNHR₅ ; R₃ est H, un radical alkyle, aralkyle ou aryle ayant jusqu'à 19 atomes de carbone ; et R₄ et R₅ sont chacun indépendamment H, OH, un radical alkyle, aralkyle, aryle, ou acyle ayant jusqu'à 19 atomes de carbone, et acide glycolique, dans laquelle la composition comprend jusqu'à 10 pour cent en poids d'acide glycolique.

2. Composition topique selon la revendication 1, comprenant 1 à 8 pour cent en poids d'acide glycolique.

3. Composition topique selon la revendication 1 ou la revendication 2, dans laquelle le dérivé de dipeptide est choisi dans le groupe constitué de N-Ac-Val-Ala-NH₂, N-Ac-Val-Ala-OH, N-Ac-Val-Ala-NHOH, N-Pr-Val-Ala-NH₂, et N-Pr-Val-Ala-OH, dans lesquels Ac est acétyle et Pr est propanoyle.

4. Composition topique selon l'une quelconque des revendications 1 à 3, dans laquelle le dérivé de N-acyldipeptide est N-Ac-Val-Ala-NH₂ dans lequel Ac est acétyle.

5. Composition topique selon l'une quelconque des revendications 1 à 4, comprenant 0,01 à 2 pour cent en poids de N-Ac-Val-Ala-NH₂ dans lequel Ac est acétyle.

6. Composition topique selon l'une quelconque des revendications 1 à 5, comprenant en outre un support topique cosmétiquement acceptable.

7. Composition topique selon la revendication 6, dans laquelle le support comprend un ou plusieurs composés parmi le dilaurate de glycéryle, le stéareth-10 et le glycérol.

8. Composition topique selon la revendication 6 ou la revendication 7, dans laquelle le support comprend de l'eau.

9. Composition topique selon l'une quelconque des revendications 6 à 8, dans laquelle le support comprend de l'eau, du dilaurate de glycéryle, du stéareth-10 et du glycérol.

10. Composition topique selon l'une quelconque des revendications précédentes, comprenant 0,01 à 2 pour cent en poids de N-Ac-Val-Ala-NH₂ dans laquelle Ac est acétyle, 1 à 8 pour cent en poids d'acide glycolique, et un support topique cosmétiquement acceptable.

11. Composition topique comprenant un dérivé N-acyldipeptide de formule :
R₁-Val-Ala-R₂
ou un isomère ou un sel de celui-ci, dans laquelle Val est la valine, Ala est l'alanine, R₁ est un radical acyle ayant jusqu'à 19 atomes de carbone ; R₂ est OR₃, NHR₄, ou NHNHR₅ ; R₃ est H, un radical alkyle, aralkyle ou aryle ayant jusqu'à 19 atomes de carbone ; et R₄ et R₅ sont chacun indépendamment H, OH, un radical alkyle, aralkyle, aryle, ou acyle ayant jusqu'à 19 atomes de carbone, et un support topique cosmétiquement acceptable comprenant du dilaurate de glycéryle, du stéareth-10, et du glycérol.

12. Procédé non thérapeutique de traitement de signes du vieillissement cutané, comprenant l'application topique sur la peau ayant besoin d'un traitement pour au moins un signe de vieillissement cutané d'une composition topique comprenant un dérivé N-acyldipeptide de formule :
R₁-Val-Ala-R₂
ou un isomère ou un sel de celui-ci, dans laquelle Val est la valine, Ala est l'alanine, R₁ est un radical acyle ayant jusqu'à 19 atomes de carbone ; R₂ est OR₃, NHR₄, ou NHNHR₅ ; R₃ est H, un radical alkyle, aralkyle ou aryle ayant jusqu'à 19 atomes de carbone ; et R₄ et R₅ sont chacun indépendamment H, OH, un radical alkyle, aralkyle, aryle, ou acyle ayant jusqu'à 19 atomes de carbone, et acide glycolique, dans laquelle la composition comprend jusqu'à 10 pour cent en poids d'acide glycolique.

13. Procédé non thérapeutique selon la revendication 12, dans lequel le signe de vieillissement cutané est choisi dans le groupe constitué des lignes, des ridules, des rides, de la perte d'élasticité, d'une peau irrégulière, des taches, de la diminution de l'épaisseur de la peau et de la synthèse anormale ou diminuée de collagène, de glycosaminoglycanes, de protéoglycanes ou d'élastine.
